# EUROPEAN PATENT APPLICATION

(11) **EP 4 825 313 A1**
(43) Date of publication of application: **21.10.2026**
(21) Application number: 25472012.1
(22) Date of filing: 16.04.2025
(51) Int. Cl.: A61B 5/107, A61B 5/11, A61B 5/00

(54) **A DEVICE AND A METHOD FOR PROVIDING BIOFEEDBACK FOR CORRECTION OF WALKING ACTIVITY**

(71) Applicant: Intelectronics Ltd, 1612 Sofia (BG)
(72) Inventor: Mahdaoui, Sabri, Sofia (BG)
(74) Representative: Benatov, Samuil Gabriel

(57) **Abstract**

The technology described in the invention relates generally to systems and methods for detecting and providing feedback to a user regarding a user's gait during walking. The device is based on measuring the impact force and knee angle to provide feedback to a subject for retraining a gait.

## Description

### FIELD OF APPLICATION

The technology described herein relates generally to systems and methods for detecting and providing feedback to a user regarding a user's gait during walking.

### PRIOR ART

Human gait has been studied at length by researchers and health specialist.

In a mapping review published from Van Gedler in 2018 with title "The use of biofeedback for gait retraining: A mapping review", 173 studies were included. The most common feedback mode used was visual feedback (42%, n=73) and the majority fed-back kinematic parameters (36%, n=62). Most studies focused on running gait retraining. Running is more studied than walking in healthy subjects as people are more prone to sustain "visible" injuries from such activity.

Research about human walk is mostly centred and focused on groups of people with pathologies. Research about running involves healthy subjects and focuses on improving performance.

In both cases, researchers have investigated extensively the use of wearables and other laboratory devices to measure a subject's gait characteristic and influence him/her to adopt a different/more optimal gait, through biofeedback, including visual, sound, and haptic cues.

There is a tendency for a growing group of people walking more and more for transportation or for leisure. Many of them are not able to find the right way to walk and wonder what they are doing wrong, as they feel that they are doing something wrong.

So, it is commonly studied how to enhance people's gait either while running of healthy subjects or on walking gait retraining for subjects with motor problems. Retraining of healthy subjects' gait is very rarely studied and as such there is a need of improvement for providing means to be used for retraining people's gait while walking.

Two recent patents relate to such devices for providing feedback for gait retraining.

US-8581731-B2 discloses an invention to retrain walking/running gait by measuring impact force and vectors associated with gait characteristics and providing visual or sound feedback. More precisely: various embodiments of the invention provide an electronic virtual running/walking coach that notifies a person as to how hard the person's feet have been impacting a surface, notifies the person as to whether the person's feet are impacting a surface at a proper relative distance from the body and/or at a proper side-to-side angle, and/or notifies the person as to how much cushioning life remains in a pair of shoes. The invention includes an informational display which provides coaching to a runner/walker, when receiving analysed information from foot impact monitoring system with a sensor. The foot impact monitoring system senses when a preset threshold level is reached and signalises to the display by sending signal from a transmitter circuit of the sensor to a receiver circuit in the display and sound device playing specific sound indicating how much impact occurred from the foot striking the surface, and that an adjustment of the gait is required. Used sensors in the impact measurement system can be an accelerometer to measure impact from the foot striking the surface while the person is walking/running, a force transducer to measure force from the foot striking the surface while the person is walking/running, a tilt sensor to measure angle/tilt of the shoe/foot/leg when a shoe impacts a surface. The sensors can be located in discrete packages that are connected to a shoe, or it may include a plurality of sensor elements that are spaced apart on a shoe to measure characteristics of the impact as the shoe rolls forward after impacting a surface or to measure characteristics of the impact as the shoe rolls inward/outward (e.g., due to pronation/supination) after impacting the surface. Accordingly, impact sensors may be spaced apart at locations in a heal portion and midfoot/forefoot location of the shoe, and/or impact sensors may be spaced apart at locations in a right and left portion of the shoe. WO2020047179A1 discloses a system for providing feedback to an individual using two devices that measure two characteristics of gait while walking, running, sprinting or during any other activity, such as ground force reaction and tibial acceleration.

Used gait characteristics are focused on two combinations of measured parameters:
- the first motion characteristic is force vector angle of ground contact and the second motion characteristic is ground contact force.
- the first motion characteristic is ground contact force and the second motion characteristic is ground contact time.

For said measurements are used sensors such as accelerometers, gyroscopes, pressure or force sensors, or location sensors. Used sensors transmit signal for reached preset value and transmit it to receivers signalling means which are used to indicate to a user that the gait is incorrect.

There is a need in the state of the art to provide more and different means for providing feedback on people's gait, so an optimal correction can be made by a subject in need of it.

### SUMMARY OF THE INVENTION

Further in the disclosure, a proper walking or proper gait of a subject refers to an efficient or biomechanically sound walking, which involves a coordinated movement pattern that maximizes efficiency, minimizes stress on the body, and promotes optimal health. It involves walking with mindfulness, paying attention to body alignment, minimizing stress on joints and muscles, and maximizing energy efficiency. Proper walking promotes optimal musculoskeletal health, reduces the risk of injury, and enhances overall well-being.

For proper walking is considered to land on the heel and roll smoothly through to the toes with each step. Ensure a heel-to-toe transition, with the weight distributed evenly across the foot.

Specific parameters and values of proper walking are not mentioned in the invention as they can vary depending on the specialists, and are thus adjusted according to each subject.

An object of the invention is to provide an alternative solution for providing feedback for people's gait, that is simple, and highly-efficient and most importantly focused on recognizing knee patterns in gait cycle, which affect the overall proper walking.

Said purpose is achieved by present invention, which relies on the measurement and analysis of two gait characteristics, namely impact force on landing and knee angle. Based on the results of the analysis, feedback is provided to the user. The measurement of the two characteristics can be direct or rely on estimations.

The device for providing biofeedback for correction of walking activity, according to the invention, comprises a supporting element, on which at least one sensor is placed. The at least one sensor is for impact force and for knee angle. The at least one sensor is connected through an electronic bus to a controller unit, comprised of a microprocessor, a battery, and a power button, wherein the controller unit is adapted to send commands for emitting signals to a signal feedback device.

The sensors for impact force and for knee angle can be separate sensors. The sensor for impact force can be a pressure sensor or at least one accelerometer or a combination of accelerometer-gyroscope or a combination of accelerometer-gyroscope-magnetometer. The sensor for knee angle can be a combination of accelerometer-gyroscope or a combination of accelerometer-gyroscope-magnetometer or a stretch sensor, or a goniometer.

Alternatively, the sensors for impact force and for knee angle can be the same sensor, which is preferably an accelerometer.

In a preferred embodiment of the invention, the signal feedback device is a haptic device.

In a preferred embodiment of the invention, the supporting element is selected from a pair of pants or leggings, or an ankle strap, or an insole fitted into a shoe, or a knee brace, or a shoe, in which the sensors and the controller unit 101 are integrated.

In a preferred embodiment of the invention, the signal feedback device is separated from the supporting element mobile device, and the controller unit and the signal feedback device each include a Bluetooth module for connection to each other.

In a preferred embodiment of the invention, it is attached or integrated into a treadmill, which is configured to measure impact force during walking activity and to provide a feedback to the user to reduce impact force, and to avoid the double bounce.

A method of providing biofeedback for correction of walking activity is created, according to the invention, comprising the steps of:
- measuring from a sensor for impact force direct or estimate ground impact data;
- measuring from a sensor for knee angle direct or estimate knee angle data;
- transmitting by the sensor for impact force and the sensor for knee angle data to a microprocessor;
- comparing by the microprocessor the ground impact value to a stored preset target value;
- analysing by the microprocessor the knee angle data and identifying existence or missing of double bounce in the gait;
- transmitting by the microprocessor to first signal means a first feedback signal if the ground impact value reaches stored target value and/or a second feedback signal if a double bounce in the gait cycle is identified;
- emitting informative signals from the first and/or second signal means indicating the transmitted feedback from the microprocessor.

In a preferred embodiment of the method, the first and the second signal means are the same means.

In a preferred embodiment of the method, the sensor for knee angle detects the existence of double bounce of a leg by measuring flexing and number of extends of a knee during a gait cycle.

In a preferred embodiment of the method, the sensor for knee angle detects the angle between the tibia and the vertical or the evolution of the angle between the femur and the vertical.

In a preferred embodiment of the method, the sensor for knee angle detects double bounce by comparing a first measured gait cycle with subsequent gait cycles.

In a preferred embodiment of the method, the sensor for knee angle is measured by processing data from two sensors on the same leg, located one above the other, obtaining the angle of the lower sensor with the vertical on the one side, obtaining the angle of the upper sensor with the vertical on the other side, and subtracting the two of them.

The present invention is a simple device to help users adopt a more elegant and smoother gait. It is based on direct study results and analysis of human movement and integrates a simple technological solution to implement the underlying observations. It assists people to walk smoother and lighter and healthier.

### DESCRIPTION OF THE FIGURES

Further in the description, the device and method for providing biofeedback for correction of walking activity of the invention are explained by a preferred embodiments, given as non-limiting the scope of the invention examples, with reference to the attached figures, where:
Figure 1 shows a diagram of the impact force as measured by an accelerometer placed on the tibia of a subject during walking activity.
Figure 2 shows a diagram of the knee angle of a subject during walking activity.
Figure 3 shows a legging with two accelerometers and a control unit.
Figure 4 shows an ankle strap comprising all elements accelerometer, microprocessor, battery, haptic feedback device.
Figure 5 shows an insole with an optional ankle strap.
Figure 6 shows a knee band with either two accelerometers, or with a stretch sensor and an accelerometer, all connected to a control unit.
Figure 7 shows a knee brace fitted with a stretch sensor.
Figure 8 shows a shoe with an accelerometer and an optional insole.

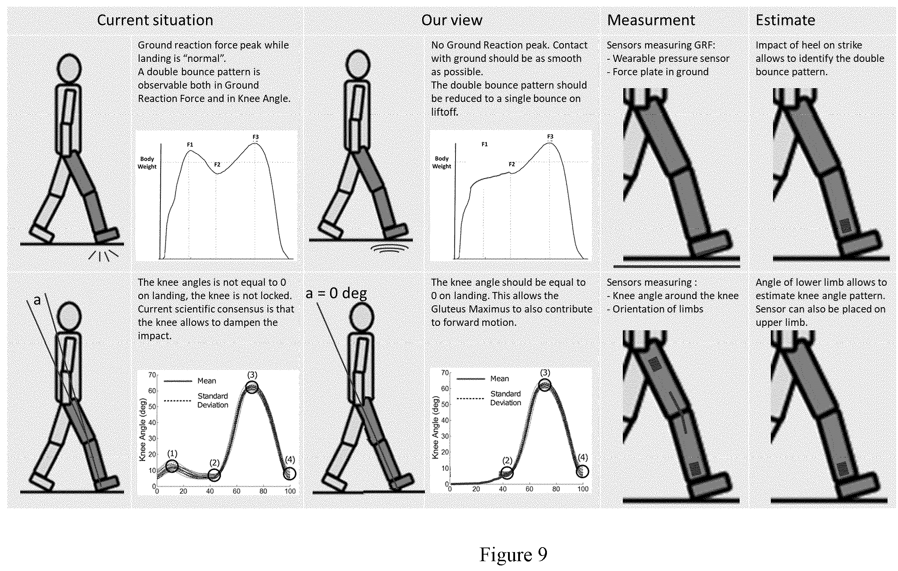

Figure 9 shows different stages of use of the invention.

### EXEMPLARY EMBODIMENT OF THE INVENTION

Impact on landing is the action of the foot coming forcibly into contact with the ground. In the case of walking, the part of the foot that usually first comes into contact with the ground is the heel. Following this fact, tibial acceleration can be a good estimation of the impact of the foot, as the shock wave can propagate directly up.

In state of the art, the gait characteristic impact on landing has been extensively studied. However, almost all the studies revolved around the running activity. One hypothesis of the present invention, is that the impact on landing is one of the most crucial characteristics for the walking activity. It should be one of the areas of focus to retrain a person to walk "properly".

To measure impact force, either a pressure sensor can be used or an accelerometer placed on the tibia. Experts in the field refer to this as vertical tibial acceleration. There is strong correlation between the two. Estimation of the impact force can also be derived from an accelerometer placed higher along the leg, even higher than the knee or on the hips.

The analysis of the impact force is straightforward: above a certain threshold value, the system considers that the user is not walking "properly" and provides feedback in the form of signal, such as vibration.

The second very important characteristic to focus on in order to efficiently train a person to walk again is the knee angle. This gait characteristic represents the angle between the femoral bone and the tibial bone, during the gait cycle.

The knee angle is important for people's gait because it directly affects the biomechanics of walking and running, in details it affects:
- Stability and Balance: The knee angle influences the stability and balance of a person while walking or running. A proper knee angle helps distribute the body's weight efficiently and maintain balance with each step;
- Efficiency of Movement: The knee angle affects the efficiency of movement during walking or running. Proper alignment of the knee joint allows for smooth and coordinated motion, minimizing energy expenditure and preventing fatigue;
- Prevention of Injuries: Incorrect knee alignment can increase the risk of various injuries, such as knee pain, ligament strains, and cartilage damage. Maintaining an appropriate knee angle helps reduce the strain on the knee joint and surrounding structures, thus lowering the risk of overuse injuries;
- Functional Range of Motion: The knee angle determines the functional range of motion of the lower limb. Adequate knee flexion and extension are essential for performing daily activities, such as walking, climbing stairs, and squatting, without discomfort or limitation.

In summary, the knee angle plays a crucial role in the biomechanics of human locomotion, affecting stability, efficiency of movement, injury prevention, and functional range of motion. Proper alignment and control of the knee joint are essential for maintaining optimal gait mechanics and overall musculoskeletal health.

During walking, the planted leg bounces twice before swinging into the next step. That is, the knee bends and extends once when the foot first touches down at the moment of a ground reaction force (GRF) peak is observable, then again just before take-off (liftoff). That first bounce helps the foot to absorb the impact of the weight as the subject hits the ground, i.e., it is used for shock absorption. The first bounce is not the natural way of walking, and is most often dictated by the use of heel-elevated shoes or other issues, and it must be avoided. So, the contact with the ground should be as smooth as possible, and the double bounce pattern should be reduced to a single bounce on liftoff.

Measurement of the knee angle can be made by pattern recognition, wherein the pattern to two types of patterns are compared, one where there are two bounces and one where this is only one. The measurement can be conducted directly by measuring the knee angle around the knee or the orientation of limbs: either through a goniometer placed directly around the knee joint, or through at least one accelerometer, or through an accelerometer and a gyroscope, or through an accelerometer, a gyroscope and a magnetometer placed below and above the knee, or through a stretch sensor placed above the knee and measuring its extension, or through a pressure sensor. The stretch sensor is not as reliable as the other solutions, but the data that can be collected, allows to determine if during the gait cycle, the knee flexes and extends twice. There is also a possibility to not directly calculate, but to estimate the knee angle by measuring the evolution of the angle between the tibia and the vertical. Similarly, there is the possibility to estimate the knee angle based on the evolution of the angle between the femur and the vertical. This is made by an accelerometer or by a combination of an accelerometer and a gyroscope. The sensors can be placed on lower or on upper limb or on both of them.

The analysis of the knee angle is made by detecting the double bounce of a leg: the first bounce corresponds to the dampening of the impact during landing, the second bounce corresponds to the extension of the knee to propel the body forward.

The simplest way to detect double bounce is to firstly identify the data corresponding to one gait cycle. There are readily available algorithms to achieve this. Once a time series knee angle as a function of time for a certain step is recorded and identified, a simple analysis of the values of the knee angle during the first half of the cycle is possible. If the value is constant, this means that the knee is locked, and therefore, no double bounce is happening. In this case, the Gluteus Maximus muscle also contributes to forward motion. If, however, the knee angle is not constant on landing, but there is a dip (occurring in increase of value then decrease of value then increase of value again) the knee is not locked and the knee allows to dampen the impact, meaning that correction of the gait is required.

Another way of determining double bounce is by studying the variations of the time series. If the knee angle increases twice and decreases twice during the cycle, then there is a double bounce. The user should be notified. If the knee angle increases only once and decreases only once, then there is no double bounce. This is one simple and power efficient way of identifying the double bounce. Other ways to identify the double bounce during a gait cycle include correlation functions, machine learning, etc.

Based on the results from the analysis of the device for providing biofeedback for correction of walking activity, according to the invention, there are four cases possible:
- The impact force is under the threshold and the knee angle does not undergo a double bounce;
- The impact force is over the threshold and the knee angle does not undergo a double bounce;
- The impact force is under the threshold and the knee angle undergoes a double bounce;
- The impact force is over the threshold and the knee angle undergoes a double bounce.

In case of an impact force over the threshold, a signal feedback device located preferably around the malleolus, would signal to the walker. For example, a haptic feedback device located preferably around the malleolus, would signal to the walker.

In case of a double bounce, the same signal feedback device would signal to the user. For example, a haptic feedback device or another haptic feedback device would signal to the user with a different type of vibration. For instance, the impact feedback could be a short loud vibration signal while the double bounce feedback could be three long and quieter vibration signals. Any combinations of vibrations signals are envisioned.

Haptic feedback is the preferred way of signalling to the user with regards to the impact force, as both rely on the tactile sense, but alternatively, also, other signals such as sound or visual can be used. Concerning the double bounce feedback, also, it might be in the form of vibration, sound, or visual.

Various embodiments are envisioned for the invention.

Both the sensor for impact force and the sensor for knee angle are inertial measurement units (IMU) or miniature inertial measurement units (MIMU) in the form of an integrated circuit, which can be with very small size such as 3mm by 3mm by 1m, or with other size, and incorporates sensing devices such as accelerometer, gyroscope, magnetometer, stretch sensor, and goniometer, depending on the specific embodiment.

An IMU can encomprise accelerometer or gyroscope or both. A MIMU encomprises accelerometer and/or gyroscope and magnetometer.

In short, the process for providing biofeedback for correction of human gait, according to the invention, works as either one common sensor for impact force 100 and knee angle 103, or two separate sensors, one for the impact force 100 and second for the knee angle 103 are connected through an electronic bus 104 to a controller unit 101, comprised of a microprocessor (also called processor or microcontroller), a battery, and a power button. When some of the sensors detects an incorrect walk, it signalizes to the controller unit 101, which is adapted to send commands for emitting signals to a signal feedback device 102, thus the subject to be aware that a correction of the gait is required.

The analysis of the impact force on landing is made by detecting if it reaches a preset threshold value.

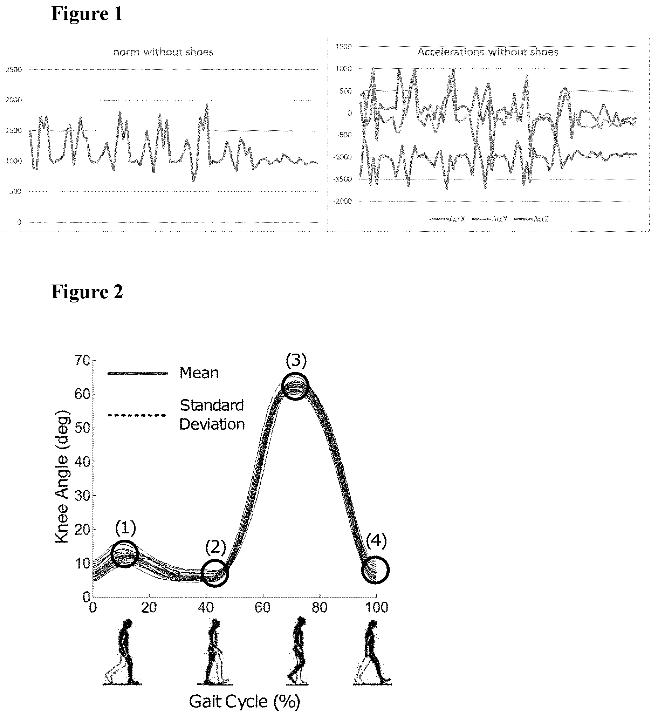

The analysis of the knee angle is made by detecting the double bounce (fig. 2) of a leg: the first bounce corresponds to the dampening of the impact during landing, the second bounce corresponds to the extension of the knee to propel the body forward.

An estimate of the variation of the knee angle can be made with only one accelerometer. It is not a direct and precise measurement of the knee able, but it allows to estimate if the knee angle has done the double bounce or not.

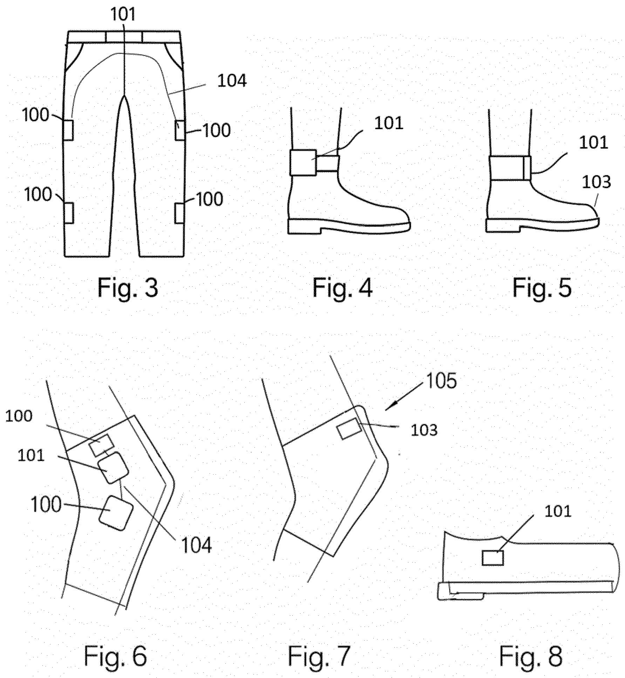

For more precise estimations, the following embodiments are provided:
In a first embodiment, shown on fig. 3, four accelerometers are used, one of which is used for sensing impact force, and for measuring the knee angle the other three accelerometers are used or alternatively accelerometer-gyroscope or accelerometer-gyroscope-magnetometer can be used for said measurement. All used sensing means 100, 103 are integrated in a pair of pants or leggings. They are connected through an electronic bus 104 to a controller unit 101 comprised of a microprocessor, a battery, a Bluetooth module, a power button. The sensors at the ankle level measure the impact force. The knee angle is measured by processing the data from two sensors on the same leg, obtaining the angle of the lower sensor with the vertical on the one side, obtaining the angle of the upper sensor with the vertical on the other side, and subtracting the two. Vibration can be either dispensed within the sensors, which then integrate vibrating device and driver for vibrating device, or within the control unit which integrates vibrating device and driver for vibrating device.

In a second embodiment, shown on fig.4, a controller unit 101 comprised of a microprocessor, a battery, a Bluetooth module, a power button and an accelerometer 100 and accelerometer-gyroscope or accelerometer-gyroscope-magnetometer 103 are placed on an ankle strap. The sensor measures impact force. It is also possible to derive from the sensor data estimating the knee angle. Thus, the knee angle data can be extrapolated from two accelerometers - one accelerometer can estimate impact force, and the same accelerometer combined with another device can allow estimation of knee angle. By processing both gait characteristics, the device can provide haptic feedback to the wearer.

In a third embodiment, shown on fig. 5, an insole fitted with a pressure sensor is inserted into a shoe. The sensor is connected via bus 104 to a controller unit 101 comprised of a microprocessor, a battery, a Bluetooth module, a power button, an accelerometer 100 as sensor for measuring impact force, and accelerometer-gyroscope or accelerometer-gyroscope-magnetometer sensors 103. In this embodiment the sensor for knee angle includes the pressure sensor and the accelerometer-gyroscope or accelerometer-gyroscope-magnetometer sensors. So, to measure knee angle, either the data from the pressure sensor can be used to estimate the occurrence of double bounce, or the pressure sensor data can be combined with data coming from the accelerometer-gyroscope or accelerometer-gyroscope-magnetometer sensors.

In a fourth embodiment, shown on fig.6, a knee brace is fitted with two accelerometers or accelerometer-gyroscope or accelerometer-gyroscope-magnetometer mounted one above the other. One of the accelerometers is used as sensor for impact force 100. The sensors are connected via bus 104 to a controller unit 101. The lower sensor measures the impact force. The knee angle is measured by processing the data from the two sensors, obtaining the angle of the lower sensor with the vertical on the one side, obtaining the angle of the upper sensor with the vertical on the other side, and subtracting the two. Vibration can be either dispensed within the sensors, which then integrate vibrating device and driver for vibrating device, or within the control unit which integrates vibrating device and driver for vibrating device.

In a fifth embodiment, shown on fig.7, a knee brace is fitted with a stretch sensor. The stretch sensor is connected to a controller unit 101. The controller unit 101 has an accelerometer 100 that allows to measure the impact force. The knee angle estimate is measured with the stretch sensor 103. Variations in the length of the stretch sensor allow to determine the occurrence of knee angle double bounce.

In a sixth embodiment, shown on fig. 8, a shoe is fitted with a controller unit 101 which may or may not comprise an accelerometer or accelerometer-gyroscope or accelerometer-gyroscope-magnetometer. The shoe also includes a sole with a pressure sensor. The impact force and the knee angle are derived from the values measured by the pressure sensor (with or without the accelerometer data).

The sensors 100, 103 can be integrated with the signal feedback device 102. Alternatively, the signal feedback device 102 can be separated from the supporting element 105 mobile device. In this case the controller unit 101 and the signal feedback device 102 each include a Bluetooth module for connection to each other.

The electronic bus 104 can be I2C, SPI or another type known from the state of the art.

It will be clear to those skilled in the art that various modifications of the device and the method for providing biofeedback for correction of walking activity are possible, which are also within the scope of the invention as defined in the appended claims. All parts of the device can be replaced with technically equivalent elements.

The reference numbers of the technical features are included in the claims solely for the purpose of increasing the comprehensibility of the claims and, therefore, these reference numbers do not have any limiting effect on the interpretation of the elements indicated by these reference numbers.

## Claims

1. A device for providing biofeedback for correction of walking activity, comprising a supporting element 105, on which at least one sensor is placed, **characterized in that** the at least one sensor is for impact force 100 and for knee angle 103, which at least one sensor is connected through an electronic bus 104 to a controller unit 101, comprised of a microprocessor, a battery, and a power button, wherein the controller unit 101 is adapted to send commands for emitting signals to a signal feedback device 102.

2. A device for providing biofeedback for correction of walking activity according to claim 1, **characterized in that** the sensors for impact force 100 and for knee angle 103 are separate sensors.

3. A device for providing biofeedback for correction of walking activity according to claim 1, **characterized in that** the sensors for impact force 100 and for knee angle 103 are the same sensor, which is an accelerometer.

4. A device for providing biofeedback for correction of walking activity according to any of the preceding claims, **characterized in that** the signal feedback device 102 is a haptic device.

5. A device for providing biofeedback for correction of walking activity according to claims 1-2, **characterized in that** the sensor for impact force 100 is a pressure sensor or at least one accelerometer or a combination of accelerometer-gyroscope or a combination of accelerometer-gyroscope-magnetometer.

6. A device for providing biofeedback for correction of walking activity according to claims 1, 2, 4 and 5, **characterized in that** the sensor for knee angle 103 is a combination of accelerometer-gyroscope or a combination of accelerometer-gyroscope-magnetometer or a stretch sensor, or a goniometer.

7. A device for providing biofeedback for correction of walking activity according to any of the preceding claims, **characterized in that** the supporting element 105 is selected from a pair of pants or leggings, or an ankle strap, or an insole fitted into a shoe, or a knee brace, or a shoe, in which the sensors 100, 103 and the controller unit 101 are integrated.

8. A device for providing biofeedback for correction of walking activity according to any of the preceding claims, **characterized in that** the signal feedback device 102 is separated from the supporting element 105 mobile device, and the controller unit 101 and the signal feedback device 102 each include a Bluetooth module for connection to each other.

9. A device for providing biofeedback for correction of walking activity according to any of the preceding claims, **characterized in that** it is attached or integrated into a treadmill.

10. A method of providing biofeedback for correction of walking activity, comprising the steps of:
- measuring from a sensor for impact force 100 direct or estimate ground impact data;
- measuring from a sensor for knee angle 103 direct or estimate knee angle data;
- transmitting by the sensor for impact force 100 and the sensor for knee angle 103 data to a microprocessor;
- comparing by the microprocessor the ground impact value to a stored preset target value;
- analysing by the microprocessor the knee angle data and identifying existence or missing of double bounce in the gait;
- transmitting by the microprocessor to first signal means a first feedback signal if the ground impact value reaches stored target value and/or a second feedback signal if a double bounce in the gait cycle is identified;
- emitting informative signals from the first and/or second signal means indicating the transmitted feedback from the microprocessor.

11. The method of claim 10, wherein the first and the second signal means are the same means.

12. The method of claims 10-11, wherein the sensor for knee angle detects the existence of double bounce of a leg by measuring flexing and number of extends of a knee during a gait cycle.

13. The method of claims 10-11, wherein the sensor for knee angle detects the angle between the tibia and the vertical or the evolution of the angle between the femur and the vertical.

14. The method of claims 10-11, wherein the sensor for knee angle detects double bounce by comparing a first measured gait cycle with subsequent gait cycles.

15. The method of claims 10-11, wherein the sensor for knee angle is measured by processing data from two sensors on the same leg, located one above the other, obtaining the angle of the lower sensor with the vertical on the one side, obtaining the angle of the upper sensor with the vertical on the other side, and subtracting the two of them.
